# FASCICULE DE BREVET EUROPEEN

(11) **EP 1 868 672 B1**
(45) Date de publication et mention de la délivrance du brevet: **13.05.2009**
(21) Numéro de dépôt: 06726246.9
(22) Date de dépôt: 14.03.2006
(51) Int. Cl.: A61M 15/00, G06M 1/16

(54) **DISPOSITIF D'INDICATION DE DOSES POUR DISTRIBUTEUR DE PRODUITS FLUIDES OU PULVERULENTS**
DOSISANZEIGEGERÄT FÜR EINEN SPENDER FÜR FLÜSSIGE ODER PULVERFÖRMIGE PRODUKTE
DOSE INDICATOR DEVICE FOR FLUID OR POWDERY PRODUCT DISPENSER

(30) Priorité: 16.03.2005 FR 0550672
(43) Date de publication de la demande: 26.12.2007
(73) Titulaire: Valois SAS, 27110 Le Neubourg (FR)
(72) Inventeur: POCOCK, Andrew, Gordon, Ickleton, Cambridge CB10 1SX (GB); KAY, Stuart, Brian, William, Melbourn Cambridgeshire SG8 6DS (GB); GREENHALGH, Paul, Newport Pagnell Buckinghamshire MK16 0ED (GB); O'HARA, Wayne, Ickleton, Cambridge CB10 1SX (GB)
(74) Mandataire: CAPRI
(86) Numéro de dépôt international: PCT/FR2006/050223
(87) Numéro de publication internationale: WO 2006/097659

(56) Documents cités:
- FR-A- 2 850 192
- US-B1- 6 659 307

## Description

La présente invention concerne un dispositif d'indication de doses, ainsi qu'un dispositif de distribution de produit fluide ou pulvérulent comportant un tel dispositif d'indication de dose.

Les dispositifs d'indication de doses sont bien connus (voir documents FR-A-2 850 192 et US-B-6 659 307) pour indiquer à l'utilisateur le nombre de doses émises ou restant à émettre à partir, par exemple, d'un dispositif de distribution de produit fluide, tel qu'un inhalateur de poudre sèche ou similaire. Les dispositifs d'indication doivent satisfaire un certain nombre d'exigences. Ainsi, ils doivent être absolument fiables, c'est-à-dire indiquer chaque dose émise, pour éviter tout risque de sous-comptage et/ou de sur-comptage. Ils doivent également être capables de compter un nombre quelconque de doses, notamment un nombre relativement important de doses, tel que par exemple soixante doses. Parallèlement, ils doivent être le moins encombrant possible, pour ne pas augmenter de manière trop importante les dimensions des dispositifs de distribution auxquels ils sont associés. Ils doivent bien entendu être simples et peu compliqués à fabriquer et à assembler. Ils doivent également assurer une indication claire et bien visible des doses émises ou restant à émettre, par exemple à travers une fenêtre de visualisation prévue dans le dispositif. Les dispositifs d'indication connus présentent généralement un certain nombre d'inconvénients. Un inconvénient majeur réside dans le fait que tout en voulant maintenir une dimension globale la plus petite possible pour le dispositif d'indication, un nombre important de doses doit pouvoir être compté, par exemple soixante, ce qui a généralement pour conséquence que les nombres ou symboles indicateurs lisibles par l'utilisateur sont de très petite dimension, avec un risque que l'utilisateur ne les distingue pas bien, ce qui peut être notamment le cas lors que l'utilisateur est une personne âgée ou ayant des problèmes de vue. Selon la nature du produit fluide ou pulvérulent à distribuer, il est essentiel que l'utilisateur soit parfaitement informé du nombre de doses émises ou restant à émettre, afin d'éviter de se retrouver, par exemple, avec un appareil vide alors qu'il a une crise nécessitant l'administration rapide de médicament. Une mauvaise indication, ou une indication peu lisible et qui risque d'être mal interprétée par l'utilisateur, présente donc un risque.

La présente invention a pour but de fournir un dispositif d'indication de dose qui ne reproduit pas les inconvénients susmentionnés.

En particulier, la présente invention a pour but de fournir un dispositif d'indication de dose qui indique de manière clairement visible le nombre de doses émises ou restant à émettre.

La présente invention a aussi pour but de fournir un dispositif d'indication de dose qui soit le moins encombrant possible, et notamment dont les dimensions sont les plus petites possibles, tout en permettant l'indication d'un grand nombre de doses, par exemple une soixantaine.

La présente invention a également pour but de fournir un dispositif d'indication qui soit sûr et fiable d'utilisation, simple et peu coûteux de fabrication et d'assemblage.

La présente invention a donc pour objet un dispositif d'indication de dose, destiné à être utilisé avec un dispositif de distribution de produit fluide ou pulvérulent, notamment pharmaceutique, pour indiquer le nombre de doses émises ou restant à émettre à partir du dispositif de distribution, comprenant un premier disque rotatif et un second disque rotatif, de préférence opaque, ledit premier disque comportant des nombres et/ou symboles indicateurs disposés de manière périphérique autour de l'axe de rotation dudit premier disque rotatif selon au moins deux cercles concentriques, et ledit second disque comportant au moins une fenêtre de visualisation.

Avantageusement, ledit second disque rotatif comporte des fenêtres de visualisation coopérant successivement et/ou alternativement avec des nombres et/ou symboles indicateurs disposés sur des cercles concentriques différents.

Avantageusement, ledit second disque comporte une pluralité de fenêtres de visualisation disposées de manière périphérique autour de l'axe de rotation dudit second disque rotatif selon au moins deux cercles concentriques.

Avantageusement, le nombre de cercles concentriques de nombres et/ou symboles indicateurs sur le premier disque rotatif est identique au nombre de cercles concentriques de fenêtres de visualisation sur le second disque rotatif.

Avantageusement, ledit second disque comporte au moins un ensemble de fenêtres de visualisation, chaque ensemble comportant au moins deux fenêtres décalées à la fois radialement et périphériquement l'une par rapport à l'autre.

Avantageusement, ledit second disque comporte plusieurs ensembles de fenêtres de visualisation similaires répartis périphériquement autour de l'axe de rotation dudit second disque.

Avantageusement, les axes de rotation des premier et second disques rotatifs sont décalés l'un par rapport à l'autre.

Avantageusement, le diamètre du premier disque est supérieur au diamètre du second disque.

Avantageusement, lesdits premier et second disques rotatifs sont tangents en un point de leurs périphéries externes.

Avantageusement, ledit second disque est superposé sur ledit premier disque, lesdits deux disques coopérant avec une ouverture de visualisation fixe du dispositif de telle sorte qu'un nombre et/ou symbole indicateur respectif du premier disque est visible dans ladite ouverture de visualisation, à travers une fenêtre de visualisation respective du second disque.

Avantageusement, les positions des nombres et/ou symboles indicateurs sur le premier disque et les positions des fenêtres de visualisation sur le second disque sont prédéterminées de telle sorte qu'à chaque actionnement du dispositif, un seul nombre et/ou symbole indicateur est visible dans ladite ouverture de visualisation fixe du dispositif, à travers une seule fenêtre de visualisation du second disque.

Avantageusement, ledit premier disque comporte une première denture périphérique coopérant avec des moyens d'actionnement, adaptés à faire tourner ledit premier disque autour de son axe de rotation, et une seconde denture périphérique, ledit second disque comportant une troisième denture périphérique coopérant avec ladite seconde denture périphérique dudit premier disque, de sorte que la rotation du premier disque entraîne la rotation du second disque.

Avantageusement, chaque nombre et/ou symbole indicateur occupe sur ledit premier disque un espace ayant une surface d'au moins 8 mm², avantageusement d'au moins 10 mm², de préférence d'au moins 12 mm².

Avantageusement, ledit premier disque comporte au moins soixante nombres et/ou symboles indicateurs, avantageusement soixante trois, répartis sur trois cercles concentriques autour de l'axe de rotation du premier disque.

Avantageusement, ledit second disque comporte vingt et une fenêtres de visualisation formant sept ensembles de trois fenêtres, lesdits sept ensembles étant répartis périphériquement autour de l'axe de rotation du second disque, lesdites trois fenêtres de chaque ensemble étant décalées à la fois radialement et périphériquement les unes par rapport aux autres.

Avantageusement, ledit premier disque a un diamètre d'environ 55 mm et/ou le second disque a un diamètre d'environ 45 mm.

Avantageusement, chaque actionnement du dispositif entraîne une rotation du premier disque d'un angle α et une rotation simultanée du second disque d'un angle 3α/2.

Avantageusement, l'angle α est égal à 22,86° et l'angle 3α/2 est égal à 34,28°.

La présente invention a également pour objet un dispositif de distribution de produit fluide ou pulvérulent comportant un dispositif d'indication de doses tel que décrit ci-dessus.

Avantageusement, le dispositif d'indication est actionné à chaque actionnement du dispositif de distribution de produit fluide.

Ces caractéristiques et avantages et d'autres de la présente invention apparaîtront plus clairement au cours de la description détaillée suivante d'un mode de réalisation avantageux de celle-ci, faite en référence aux dessins joints, donnés à titre d'exemples non limitatifs, et sur lesquels,
La figure 1 est une vue schématique de dessus d'un dispositif d'indication de doses selon un mode de réalisation avantageux de la présente invention,
La figure 2 est une vue similaire à celle de la figure 1, représentant seulement le premier disque rotatif,
La figure 3 est une vue similaire à celle de la figure 2 montrant un disque rotatif connu de l'art antérieur,
La figure 4 est une vue schématique de dessous, montrant les moyens d'actionnement du dispositif d'indication, et
La figure 5 est une vue similaire à celle de la figure 1, montrant un dispositif d'indication installé dans un dispositif de distribution de produit fluide ou pulvérulent.

La présente invention concerne un dispositif d'indication de doses destiné à être utilisé avec un dispositif de distribution de produit fluide ou pulvérulent, tel qu'un inhalateur de poudre sèche, et qui permet d'indiquer à l'utilisateur le nombre de doses émises ou restant à émettre. Il est entendu que le dispositif d'indication de la présente invention s'applique à tout type de dispositif de distribution de produit fluide ou pulvérulent, de sorte que ce dispositif de distribution ne sera pas décrit en détails ci-après. Seules les parties du dispositif de distribution recevant le dispositif d'indication et les moyens d'actionnement de celui-ci seront décrites succinctement ci-après. Avec ces informations, l'homme du métier est capable d'appliquer le dispositif d'indication de la présente invention à tout type de dispositif de distribution de produit fluide ou pulvérulent de son choix.

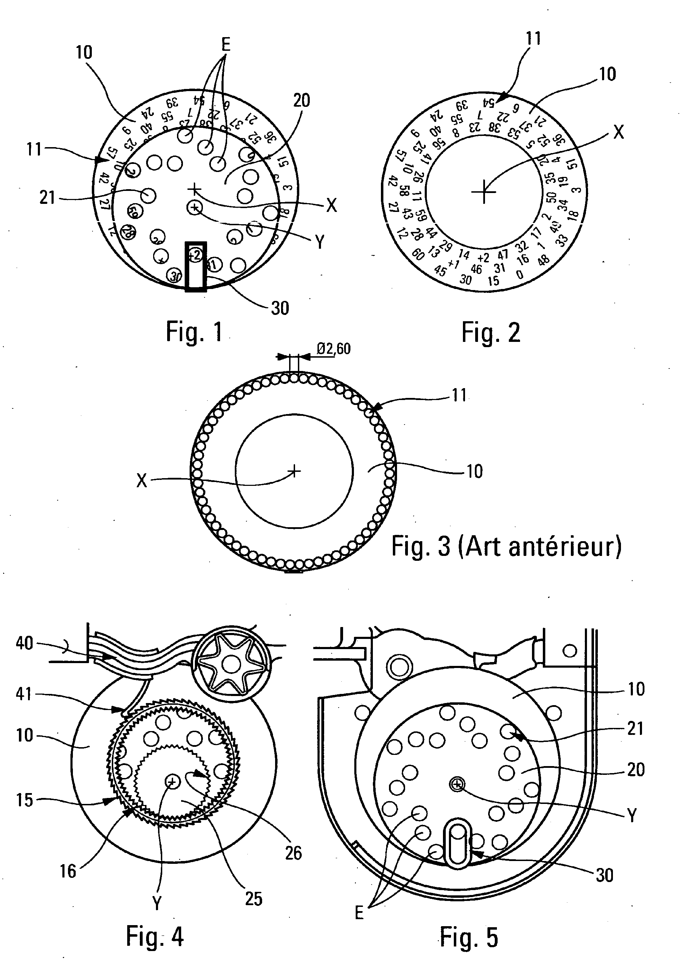

En référence aux figures, le dispositif d'indication de doses selon l'invention comprend un premier disque rotatif 10 et un second disque rotatif 20. Le second disque rotatif 20 est de préférence opaque, et il est de préférence superposé sur ledit premier disque rotatif 10. Avantageusement, l'axe de rotation X du premier disque rotatif 10 est décalé par rapport à l'axe de rotation Y du second disque rotatif 20, comme visible sur la figure 1. De plus, le second disque rotatif 20 a de préférence un diamètre inférieur au diamètre du premier disque rotatif 10. Avantageusement, les premier et second disques rotatifs 10, 20 se touchent en un point de leurs périphéries externes, comme cela est clairement visible sur les figures 1 et 5.

Le premier disque rotatif 10 comporte des nombres et/ou des symboles indicateurs 11 qui sont disposés de manière périphérique autour de l'axe de rotation X selon au moins deux cercles concentriques, de préférence trois cercles concentriques comme cela est représenté sur la figure 2. Le second disque rotatif 20 comporte au moins une, de préférence plusieurs fenêtres de visualisation 21 coopérant avantageusement successivement et/ou alternativement avec des nombres et/ou symboles indicateurs 11 disposés sur des cercles concentriques différents du premier disque rotatif 10. Cette mise en oeuvre, qui prévoit de décaler radialement les nombres et/ou symboles indicateurs permet de réaliser ceux-ci dans une plus grande dimension, et donc de manière plus lisible pour l'utilisateur. En particulier, la mise en oeuvre représentée sur les figures et qui sera détaillée plus amplement ci-après permet de réaliser des nombres et/ou symboles indicateurs pouvant occuper un espace sur le premier disque rotatif 10 d'au moins 8 mm², avantageusement d'au moins 10 mm², de préférence d'au moins 12 mm². L'exemple représenté sur les figures, notamment sur la figure 2, permet en réalité de disposer les nombres et/ou symboles indicateurs dans des cercles de 4 mm de diamètre, ce qui fait une surface de 12,56 mm², voire même dans un carré de 4 mm de côté, c'est-à-dire une surface de 16 mm². En comparaison, la figure 3 montre un disque rotatif 10 de même dimension, rotatif autour d'un axe de rotation X et comportant le même nombre de nombres et/ou symboles indicateurs 11 répartis autour de sa périphérie sur un seul cercle. Dans ce cas, chaque nombre et/ou symbole indicateur 11 occupe une surface circulaire de diamètre 2,6 mm, c'est-à-dire une surface de 5,30 mm². Au mieux, si l'on considère des carrés de 2,6 mm de côté, la surface maximale occupée par les nombres et/ou symboles indicateurs 11 du disque de la figure 3 ne dépasse pas 6,76 mm². On constate donc que la présente invention permet de réaliser les nombres et/ou symboles indicateurs de manière nettement plus grande, et donc nettement plus visible pour l'utilisateur. Les risques que l'utilisateur se trompe sur l'indication sont donc nettement réduits, voire complètement éliminés.

De préférence, le second disque indicateur 20 comporte une pluralité de fenêtres de visualisation 21 également disposées de manière périphérique autour de l'axe de rotation Y du second disque rotatif 20 selon au moins deux cercles concentriques, de préférence selon trois cercles concentriques comme représenté sur la figure 1. Avantageusement, le nombre de cercles concentriques prévus pour les nombres et/ou symboles indicateurs 11 sur le premier disque rotatif 10 est égal au nombre de cercles concentriques prévus pour les fenêtres de visualisation 21 sur le second disque rotatif 20. Bien entendu, le mode de réalisation représenté n'est qu'un exemple, et un nombre différent de cercles concentriques, par exemple deux, quatre ou plus, peut être envisagé. Avantageusement, les fenêtres de visualisation 21 du second disque rotatif 20 sont disposées selon au moins un ensemble E, chaque ensemble E comportant au moins deux fenêtres 21 décalées à la fois radialement et périphériquement l'une par rapport à l'autre. Avantageusement, plusieurs ensembles E de fenêtres de visualisation similaires sont prévus en étant répartis périphériquement autour de l'axe de rotation Y du second disque rotatif 20. Dans l'exemple représenté sur les figures 1 et 5, il y a sept ensembles E comportant chacun trois fenêtres de visualisation 21 décalées les une par rapport aux autres, à la fois radialement et périphériquement.

Le second disque rotatif 20 est donc superposé de préférence sur le premier disque rotatif 10, et les deux disques rotatifs coopèrent avec une ouverture de visualisation fixe 30 du dispositif pour afficher un nombre et/ou symbole indicateur dans ladite ouverture de visualisation 30 à travers une fenêtre de visualisation 21 du second disque rotatif 20. Les autres nombres et/ou symboles indicateurs 11 ne sont pas visibles par l'utilisateur, en étant masqués soit par le second disque opaque 20, soit par la partie du dispositif de distribution entourant l'ouverture de visualisation fixe 30. Dans l'exemple représenté sur la figure 1, c'est le symbole + et le chiffre 2 qui sont affichés dans l'ouverture de visualisation 30. En se référant à la figure 2, on constate que l'exemple représenté montre un dispositif d'indication dans lequel on décompte à partir de 60 jusqu'à 0, les deux coups (ou actionnements) avant la première utilisation réelle représentant l'amorçage et étant signalés par les indications +2 et +1, destinées à montrer à l'utilisateur que les deux premiers actionnements, susceptibles de former des doses non complètes, ou alors correspondant à des coups d'essai, ne doivent pas être inhalés. Dans ce cas, ces indications +1 et +2 peuvent par exemple être représentées dans un code couleur différent, éventuellement en vert. De manière similaire, lorsqu'on arrive vers la fin, c'est-à-dire au niveau des cinq dernières doses à émettre, les chiffres 5, 4, 3, 2, 1 et 0 peuvent également être représentés dans un code couleur différent, par exemple le rouge, pour avertir l'utilisateur que son dispositif est bientôt vide. On constate avantageusement que la répartition des nombres et/ou symboles indicateurs sur le premier disque rotatif 10 correspond sensiblement à la répartition des fenêtres de visualisation 21 sur le second disque rotatif 20, en ce que des nombres qui se suivent sont décalés à la fois radialement, c'ést-à-dire disposés sur des cercles concentriques différents, et périphériquement autour du premier disque rotatif 10.

En fonctionnement, l'un des deux disques rotatifs est entraîné en rotation lors de l'actionnement du dispositif de distribution de produit fluide. Il s'agit de préférence du premier disque rotatif 10. Des moyens de couplage sont avantageusement prévus entre le premier disque rotatif 10 et le second disque rotatif 20 pour simultanément entraîner en rotation le second disque rotatif 20. A chaque actionnement, le premier disque rotatif 10 tourne donc par rapport à l'ouverture de visualisation fixe 30 pour afficher le nombre suivant dans cette ouverture de visualisation 30, et simultanément le second disque rotatif 20 tourne également pour venir coopérer avec le nombre adéquat du premier disque rotatif tout en masquant le reste de l'ouverture de visualisation 30. En pratique, avec l'exemple de réalisation représenté comportant trois cercles concentriques de nombre 11 et de fenêtres de visualisation 21, l'affichage dans l'ouverture de visualisation 30 pour trois doses successives se fera en trois positions différentes dans ladite ouverture de visualisation fixe 30, à savoir une position supérieure, telle que représentée sur la figure 1, une position centrale et une position inférieure. La dose suivant celle affichée en position inférieure dans l'ouverture de visualisation fixe 30 sera à nouveau affichée en position supérieure. En réalité, ceci correspond aux ensembles E de fenêtres de visualisation 21 qui affichent successivement des nombres de doses qui se suivent en des positions radiales différentes dans l'ouverture de visualisation fixe 30. Lorsque l'on passe à l'ensemble E suivant, on recommence la même séquence, et ainsi de suite.

Dans l'exemple spécifique représenté sur les figures 1 et 2, comportant 63 nombres et/ou symboles indicateurs et 21 fenêtres de visualisation, et avec des premier et second disques rotatifs qui ont avantageusement un diamètre respectif d'environ 55 mm et 45 mm, chaque actionnement du dispositif entraîne une rotation du premier disque rotatif 10 d'un angle α et une rotation simultanée du second disque 20 d'un angle 3α/2. De manière plus précise, dans cet exemple de réalisation, l'angle α est égal à 22,86° et l'angle 3α/2 est égal à 34,28°. Bien entendu, avec un nombre de doses et/ou des dimensions de disques différents, la répartition des nombres et/ou symboles indicateurs peut être différente et tous les paramètres pourront alors être adaptés pour obtenir à nouveau une dimension optimale pour chaque nombre et/ou symbole indicateur inscrit sur premier disque rotatif 10. Il en est de même de la répartition des fenêtres de visualisation 21 sur le second disque rotatif 20. Un modèle mathématique permet d'optimiser les relations entre les dimensions des disques rotatifs 10, 20, le nombre de doses à indiquer et les positions et dimensions résultantes des nombres et/ou symboles indicateurs 11 sur le premier disque rotatif 10, et des fenêtres de visualisation sur le second disque rotatif 20. Une modification des formes et dimensions d'une ou des dentures 15, 16, 26 fournit aussi un paramètre réglable.

La présente invention permet donc de réaliser des nombres et/ou symboles indicateurs de plus grande dimension sur un seul disque, ce qui simplifie la fabrication, l'assemblage ainsi que le fonctionnement du dispositif d'indication, tout en assurant une information plus lisible pour l'utilisateur.

La figure 4 représente un exemple de moyens d'actionnement du dispositif d'indication de la présente invention. Dans cet exemple, une patte flexible 41 solidaire d'une partie d'actionnement 40 du dispositif de distribution de produit fluide coopère avec une première denture périphérique 15, externe dans cet exemple, prévue sur le premier disque rotatif 10. Ce premier disque rotatif 10 comporte en outre une seconde denture périphérique 16, interne dans cet exemple, qui coopère avec une troisième denture périphérique 26 prévue sur le second disque rotatif 20. Sur la figure 4, qui est une vue schématique de dessous, on constate qu'avantageusement le premier disque rotatif 10 n'est pas plein mais est au contraire vide en son centre, de sorte que la partie du second disque rotatif 20 qui comporte cette denture 26 sur sa périphérie pénètre dans cette ouverture centrale du premier disque rotatif 10 dont le bord est formé par la seconde denture 16. De cette manière, chaque fois que la patte élastique 41 entraîne en rotation le premier disque rotatif 10, celui-ci entraîne en rotation le second disque rotatif 20 par l'intermédiaire de la coopération entre la seconde denture 16 et la troisième denture 26. D'autres moyens d'actionnement sont bien entendu ainsi envisageables. Bien entendu, des moyens anti-retour et similaire peuvent être prévus pour empêcher une rotation des disques dans le sens inverse de celui imposé par les moyens d'actionnement.

Bien que la présente invention ait été décrite en référence à un mode de réalisation particulier de celle-ci, il est clair qu'elle n'est pas limitée par cet exemple, et qu'au contraire, un homme du métier peut y apporter toute modification utile sans sortir du cadre de la présente invention tel que défini par les revendications annexées.

## Revendications

1. Dispositif d'indication de dose, destiné à être utilisé avec un dispositif de distribution de produit fluide ou pulvérulent, notamment pharmaceutique, pour indiquer le nombre de doses émises ou restant à émettre à partir du dispositif de distribution, comprenant un premier disque rotatif (10), ledit premier disque (10) comportant des nombres et/ou symboles indicateurs (11) disposés de manière périphérique autour de l'axe de rotation (X) dudit premier disque rotatif (10) selon au moins deux cercles concentriques **caractérisé en ce qu'**il comprend un second disque rotatif (20), de préférence opaque, ledit second disque (20) comportant au moins une fenêtre de visualisation (21) étant superposé sur ledit premier disque (10).

2. Dispositif selon la revendication 1, dans lequel ledit second disque rotatif (20) comporte des fenêtres de visualisation (21) coopérant successivement et/ou alternativement avec des nombres et/ou symboles indicateurs (11) disposés sur des cercles concentriques différentes.

3. Dispositif selon la revendication 1 ou 2, dans lequel ledit second disque (20) comporte une pluralité de fenêtres de visualisation (21) disposées de manière périphérique autour de l'axe de rotation (Y) dudit second disque rotatif (20) selon au moins deux cercles concentriques.

4. Dispositif selon la revendication 3, dans lequel le nombre de cercles concentriques de nombres et/ou symboles indicateurs (11) sur le premier disque rotatif (10) est identique au nombre de cercles concentriques de fenêtres de visualisation (21) sur le second disque rotatif (20).

5. Dispositif selon l'une quelconque des revendications précédentes, dans lequel ledit second disque (20) comporte au moins un ensemble (E) de fenêtres de visualisation (21), chaque ensemble (E) comportant au moins deux fenêtres (21) décalées à la fois radialement et périphériquement l'une par rapport à l'autre.

6. Dispositif selon la revendication 5, dans lequel ledit second disque (20) comporte plusieurs ensembles (E) de fenêtres de visualisation (21) similaires réparties périphériquement autour de l'axe de rotation (Y) dudit second disque (20).

7. Dispositif selon l'une quelconque des revendications précédentes, dans lequel les axes de rotation (X, Y) des premier et second disques rotatifs (10, 20) sont décalés l'un par rapport à l'autre.

8. Dispositif selon l'une quelconque des revendications précédentes, dans lequel le diamètre du premier disque (10) est supérieur au diamètre du second disque (20).

9. Dispositif selon la revendication 8, dans lequel lesdits premier et second disques rotatifs (10, 20) sont tangents en un point de leurs périphéries externes.

10. Dispositif selon l'une quelconque des revendications précédentes, dans lequel ledit second disque (20) est superposé sur ledit premier disque (10), lesdits deux disques (10, 20) coopérant avec une ouverture de visualisation fixe (30) du dispositif de telle sorte qu'un nombre et/ou symbole indicateur respectif (11) du premier disque (10) est visible dans ladite ouverture de visualisation (30), à travers une fenêtre de visualisation respective (21) du second disque (20).

11. Dispositif selon la revendication 10, dans lequel les positions des nombres et/ou symboles indicateurs (11) sur le premier disque (10) et les positions des fenêtres de visualisation (21) sur le second disque (20) sont prédéterminées de telle sorte qu'à chaque actionnement du dispositif, un seul nombre et/ou symbole indicateur (11) est visible dans ladite ouverture de visualisation fixe (30) du dispositif, à travers une seule fenêtre de visualisation (21) du second disque (20).

12. Dispositif selon l'une quelconque des revendications précédentes, dans lequel ledit premier disque (10) comporte une première denture périphérique (15) coopérant avec des moyens d'actionnement (40, 41), adaptés à faire tourner ledit premier disque (10) autour de son axe de rotation (X), et une seconde denture périphérique (16), ledit second disque (20) comportant une troisième denture périphérique (26) coopérant avec ladite seconde denture périphérique (16) dudit premier disque (10), de sorte que la rotation du premier disque (10) entraîne la rotation du second disque (20).

13. Dispositif selon l'une quelconque des revendications précédentes, dans lequel chaque nombre et/ou symbole indicateur (11) occupe sur ledit premier disque (10) un espace ayant une surface d'au moins 8 mm², avantageusement d'au moins 10 mm², de préférence d'au moins 12 mm².

14. Dispositif selon l'une quelconque des revendications précédentes, dans lequel ledit premier disque (10) comporte au moins soixante nombres et/ou symboles indicateurs (11), avantageusement soixante trois, répartis sur trois cercles concentriques autour de l'axe de rotation (X) du premier disque (10).

15. Dispositif selon l'une quelconque des revendications précédentes, dans lequel ledit second disque (20) comporte vingt et une fenêtres de visualisation (21) formant sept ensembles (E) de trois fenêtres (21), lesdits sept ensembles (E) étant répartis périphériquement autour de l'axe de rotation (Y) du second disque (20), lesdites trois fenêtres (21) de chaque ensemble (E) étant décalées à la fois radialement et périphériquement les unes par rapport aux autres.

16. Dispositif selon l'une quelconque des revendications précédentes, dans lequel ledit premier disque (10) a un diamètre d'environ 55 mm et/ou le second disque (20) a un diamètre d'environ 45 mm.

17. Dispositif selon l'une quelconque des revendications précédentes, dans lequel chaque actionnement du dispositif entraîne une rotation du premier disque (10) d'un angle α et une rotation simultanée du second disque (20) d'un angle 3α/2.

18. Dispositif selon la revendication 16, dans lequel l'angle α est égal à 22,86° et l'angle 3α/2 est égal à 34,28°.

19. Dispositif de distribution de produit fluide ou pulvérulent, **caractérisé en ce qu'**il comporte un dispositif d'indication de dose, selon l'une quelconque des revendications précédentes.

20. Dispositif selon la revendication 19, dans lequel le dispositif d'indication est actionné à chaque actionnement du dispositif de distribution de produit fluide.

## Claims

1. A dose-indicator device for using with a dispenser device for dispensing fluid or powder, in particular a pharmaceutical, so as to indicate the number ot doses that have been emitted or that remain to be emitted from the dispenser device, the indicator device comprising a first rotary disk (10) said first disk (10) including indicator numbers and/or symbols (11) that are disposed in peripheral manner about the axis of rotation (X) of said first rotary disk (10) around at least two concentric circles, **characterized in that** it comprises a second rotary disk (20) that is preferably opaque, said second disk (20) including at least one viewing window (21) being superposed on said first disk (10).

2. A device according to claim 1, in which said second rotary disk (20) includes viewing windows (21) that cooperate successively and/or alternately with indicator numbers and/or symbols (11) that are disposed around different concentric circles.

3. A device according to claim 1 or claim 2, in which said second disk (20) includes a plurality of viewing windows (21) that are disposed in peripheral manner about the axis of rotation (Y) of said second rotary disk (20) around at least two concentric circles.

4. A device according to claim 3, in which the number of concentric circles of indicator numbers and/or symbols (11) on the first rotary disk (10) is identical to the number of concentric circles of viewing windows (21) on the second rotary disk (20).

5. A device according to any preceding claim, in which said second disk (20) includes at least one set (E) of viewing windows (21), each set (E) comprising at least two windows (21) that are mutually offset both radially and peripherally.

6. A device according to claim 5, in which said second disk (20) includes a plurality of similar sets (E) of viewing windows (21) that are distributed peripherally about the axis of rotation (Y) of said disk (20).

7. A device according to any preceding claim, in which the axes of rotation (X, Y) of the first and second rotary disks (10, 20) are mutually offset.

8. A device according to any preceding claim, in which the diameter of the first disk (10) is greater than the diameter of the second disk (20).

9. A device according to claim 8, in which said first and second rotary disks (10, 20) are tangential at a point on their outer peripheries.

10. A device according to any preceding claim, in which said second disk (20) is superposed on said first disk (10), said two disks (10, 20) co-operating with a stationary viewing slot (30) of the device, such that a respective indicator number and/or symbol (11) of the first disk (10) is visible in said viewing slot (30), through a respective viewing window (21) of the second disk (20).

11. A device according to claim 10, in which the positions of the indicator numbers and/or symbols (11) on the first disk (10) and the positions of the viewing windows (21) on the second disk (20) are predetermined, such that each time the device is actuated, a single indicator number and/or symbol (11) is visible in said stationary viewing slot (30) of the device, through a single viewing window (21) of the second disk (20).

12. A device according to any preceding claim, in which said first disk (10) includes a first peripheral set of teeth (15) that co-operates with actuator means (40, 41) that are adapted to cause said first disk (10) to turn about its axis of rotation (X) and a second peripheral set of teeth (16), said second disk (20) including a third peripheral set of teeth (26) that co-operates with said second peripheral set of teeth (16) of said first disk (10), such that turning the first disk (10) causes the second disk (20) to turn.

13. A device according to any preceding claim, in which, on said first disk (10), each indicator number and/or symbol (11) occupies a space having a surface area of at least 8 mm², advantageously at least 10 mm², and preferably at least 12 mm².

14. A device according to any preceding claim, in which said first disk (10) includes at least sixty indicator numbers and/or symbols (11), advantageously sixty-three, distributed around three concentric circles about the axis of rotation (X) of the first disk (10).

15. A device according to any preceding claim, in which said second disk (20) includes twenty-one viewing windows (21) forming seven sets (E) of three windows (21), said seven sets (E) being distributed peripherally about the axis of rotation (Y) of the second disk (20), said three windows (21) of each set (E) being mutually offset both radially and peripherally.

16. A device according to any preceding claim, in which said first disk (10) has a diameter of about 55 mm and/or the second disk (20) has a diameter of about 45 mm.

17. A device according to any preceding claim, in which, each time the device is actuated, the first disk (10) is caused to turn through an angle a, and the second disk (20) is caused to turn simultaneously through an angle 3α/2.

18. A device according to claim 16, in which the angle α is equal to 22.86° and the angle 3α/2 is equal to 34.28°.

19. A fluid or powder dispenser device, **characterized in that** it includes a dose-indicator device according to any preceding claim.

20. A device according to claim 19, in which the indicator device is actuated each time the fluid dispenser device is actuated.

## Patentansprüche

1. Vorrichtung zur Anzeige einer Dosis zur Verwendung mit einer Ausgabevorrichtung für ein flüssiges oder pulverförmiges, insbesondere pharmazeutisches, Produkt zum Anzeigen der Anzahl aus der Ausgabevorrichtung ausgegebener oder noch auszugebender Dosen, aufweisend eine erste Drehscheibe (10), wobei die erste Scheibe (10) Zahlen- und/oder Symbolanzeigen (11) aufweist, die am Umfang um die Drehachse (X) der ersten Drehscheibe (10) gemäß mindestens zweier konzentrischer Kreise angeordnet sind, **dadurch gekennzeichnet, dass** sie eine zweite Drehscheibe (20), die vorzugsweise undurchsichtig ist, aufweist, wobei die zweite Scheibe (20) mindestens ein Sichtfenster (21) aufweist und der ersten Scheibe (10) überlagert ist.

2. Vorrichtung nach Anspruch 1, wobei die zweite Drehscheibe (20) Sichtfenster (21) aufweist, die nacheinander und/oder abwechselnd mit den Zahlen- und/oder Symbolanzeigen (11), die auf verschiedenen konzentrischen Kreisen angeordnet sind, zusammenwirken.

3. Vorrichtung nach Anspruch 1 oder 2, wobei die zweite Scheibe (20) eine Vielzahl von Sichtfenstern (21) aufweist, die im Umfang um die Drehachse (Y) der zweiten Drehscheibe (20) gemäß mindestens zweier konzentrischer Kreise angeordnet sind.

4. Vorrichtung nach Anspruch 3, wobei die Anzahl der konzentrischen Kreise von Zahlen- und/oder Symbolanzeigen (11) auf der ersten Drehscheibe (10) mit der Anzahl der konzentrischen Kreise von Sichtfenstern (21) auf der zweiten Drehscheibe (20) identisch ist.

5. Vorrichtung nach einem der vorhergehenden Ansprüche, wobei die zweite Scheibe (20) mindestens einen Satz (E) von Sichtfenstern (21) aufweist, wobei jeder Satz (E) mindestens zwei Fenster (21) aufweist, die sowohl radial als auch im Umfang zueinander versetzt sind.

6. Vorrichtung nach Anspruch 5, wobei die zweite Scheibe (20) mehrere Sätze (E) von ähnlichen Sichtfenstern (21) aufweist, die im Umfang um die Drehachse (Y) der zweiten Scheibe (20) verteilt sind.

7. Vorrichtung nach einem der vorhergehenden Ansprüche, wobei die Drehachsen (X, Y) der ersten und der zweiten Drehscheibe (10, 20) zueinander versetzt sind.

8. Vorrichtung nach einem der vorhergehenden Ansprüche, wobei der Durchmesser der ersten Scheibe (10) größer als der Durchmesser der zweiten Scheibe (20) ist.

9. Vorrichtung nach Anspruch 8, wobei die erste und die zweite Drehscheibe (10, 20) an einem Punkt ihres äußeren Umfangs tangierend sind.

10. Vorrichtung nach einem der vorhergehenden Ansprüche, wobei die zweite Scheibe (20) der ersten Scheibe (10) überlagert ist, wobei die beiden Scheiben (10, 20) mit einer festen Sichtöffnung (30) der Vorrichtung derart zusammenwirken, dass eine jeweilige Zahlen- und/oder Symbolanzeige (11) der ersten Scheibe (10) über ein jeweiliges Sichtfenster (21) der zweiten Scheibe (20) in der Sichtöffnung (30) sichtbar ist.

11. Vorrichtung nach Anspruch 10, wobei die Positionen der Zahlen- und/oder Symbolanzeigen (11) auf der ersten Scheibe (10) und die Positionen der Sichtfenster (21) auf der zweiten Scheibe (20) derart vorbestimmt sind, dass bei jeder Betätigung der Vorrichtung eine einzige Zahlen- und/oder Symbolanzeige (11) über ein einziges Sichtfenster (21) der zweiten scheibe (20) in der festen Sichtöffnung (30) der Vorrichtung sichtbar ist.

12. Vorrichtung nach einem der vorhergehenden Ansprüche, wobei die erste Scheibe (10) eine erste Umfangszahnung (15) aufweist, die mit Betätigungsmitteln (40, 41) zusammenwirkt, die dazu geeignet sind, die erste Scheibe (10) um ihre Drehachse (X) zu drehen, und eine zweite Umfangszahnung (16) aufweist, wobei die zweite Scheibe (20) eine dritte Umfangszahnung (26) aufweist, die mit der zweiten Umfangszahnung (16) der ersten Scheibe (10) zusammenwirkt, so dass die Drehung der ersten Scheibe (10) die Drehung der zweiten Scheibe (20) bewirkt.

13. Vorrichtung nach einem der vorhergehenden Ansprüche, wobei jede Zahlen- und/oder Symbolanzeige (11) auf der ersten Scheibe (10) einen Platz einnimmt, der eine Oberfläche von mindestens 8 mm², vorteilhafterweise von mindestens 10 mm², vorzugsweise von mindestens 12 mm² aufweist.

14. Vorrichtung nach einem der vorhergehenden Ansprüche, wobei die erste Scheibe (10) mindestens sechzig, vorteilhafterweise dreiundsechzig, Zahlen- und/oder Symbolanzeigen (11) aufweist, die auf drei konzentrischen Kreisen um die Drehachse (X) der ersten Scheibe (10) verteilt sind.

15. Vorrichtung nach einem der vorhergehenden Ansprüche, wobei die zweite Scheibe (20) einundzwanzig Sichtfenster (21) aufweist, die sieben Sätze (E) von drei Fenstern (21) bilden, wobei die sieben Sätze (E) am Umfang um die Drehachse (Y) der zweiten Scheibe (20) verteilt sind, wobei die drei Fenster (21) jedes Satzes (E) sowohl radial als auch im Umfang zueinander versetzt sind.

16. Vorrichtung nach einem der vorhergehenden Ansprüche, wobei die erste Scheibe (10) einen Durchmesser von etwa 55 mm und/oder die zweite Scheibe (20) einen Durchmesser von etwa 45 mm aufweist.

17. Vorrichtung nach einem der vorhergehenden Ansprüche, wobei jede Betätigung der Vorrichtung eine Drehung der ersten Scheibe (10) um einen Winkel α und eine gleichzeitige Drehung der zweiten Scheibe (20) um einen Winkel 3α/2 bewirkt.

18. Vorrichtung nach Anspruch 16, wobei der Winkel α gleich 22,86° und der Winkel 3α/2 gleich 34,28° ist.

19. Ausgabevorrichtung für ein flüssiges oder pulverförmiges Produkt, **dadurch gekennzeichnet, dass** es eine Vorrichtung zur Anzeige einer Dosis nach einem der vorhergehenden Ansprüche aufweist.

20. Vorrichtung nach Anspruch 19, wobei die Anzeigevorrichtung bei jeder Betätigung der Ausgabevorrichtung für ein flüssiges Produkt betätigt wird.
